# EUROPEAN PATENT SPECIFICATION

(11) **EP 2 676 571 B1**
(45) Date of publication and mention of the grant of the patent: **12.10.2016**
(21) Application number: 12746692.8
(22) Date of filing: 13.02.2012
(51) Int. Cl.: A46B 17/06, A61L 2/07, A61L 2/24

(54) **APPARATUS FOR DISINFECTING AND SANITISING BRUSHES**
VORRICHTUNG ZUR DESINFEKTION UND SANIERUNG VON BÜRSTEN
APPAREIL POUR LA DÉSINFECTION ET L'HYGIÉNISATION DE BROSSES

(30) Priority: 18.02.2011 BR PI1100260
(43) Date of publication of application: 25.12.2013
(73) Proprietor: Bio-Art Equipamentos Odontológicos Ltda, CEP 13568-000 São Cantos SP (BR)
(72) Inventor: GERMANO, José Piccin, São Cantos - SP, CEP: 13569-290 (BR)
(74) Representative: Zinkler, Franz
(86) International application number: PCT/BR2012/000041
(87) International publication number: WO 2012/109721

(56) References cited:
- BR-A- PI0 605 466
- BR-A- PI0 700 573
- BR-U2- MU8 702 451
- GB-A- 2 336 313
- US-A- 2 592 131
- US-A- 4 816 648
- US-A1- 2004 033 182
- US-B1- 6 171 559
- US-B1- 6 565 819
- US-B1- 6 565 819
- US-B2- 7 378 067

## Description

### FIELD OF THE INVENTION

The present application relates to the field of sanitizing and disinfecting apparatuses of brushes, combs, instruments and accessories in general, more specifically to an apparatus able to provide the microorganisms removal from various brushes, using the steam as action factor.

### BACKGROUND OF THE INVENTION

There are known some patent documents which disclose products of sanitizing and disinfecting brushes, combs, instruments and accessories in order to eliminate bacteria and microorganisms, preventing the transmission of capillary diseases.

The International Publication WO2005046389A1, (corresponding to IT2003T0000908) discloses an apparatus for cleaning and sanitizing brushes, where the brushes are arranged in a housing which remains in the upright position and so there is in a first step the removal of the hair present on the brush and following the contact of the steam with the brush the disinfection is performed. But the handle of the brush and comb are also in contact with the steam, so they are warmed, causing accidents. The system still has rotary movements for brushes, increasing the product price.

The Brazilian application PI0605466-8, from the same holder or applicant of the present application is a sanitizer apparatus for disinfecting and sanitizing hair brushes, brushes for veterinary use or similar instruments. However, this document presents a sanitizer which enables disinfecting a brush a time and, moreover, the handle of brushes, combs, among others, is arranged vertically to the sanitizing chamber, and it is inevitable the contact of the steam with the handle of these instruments and the consequent damage to them.

In this sense, the concept of the invention object of the present application represents a technological progress in relation to the product disclosed by the document object of the Brazilian application PI0605466-8 since it allows to perform the brushes disinfecting and sanitizing in greater amount and with brushes handles and other products to be disinfected and sanitized facing the outside of the apparatus, avoiding any heating due to the steam in plastic or wooden handles of the brushes or combs.

The document WO2010/035290, in turn, discloses an apparatus to clean only one brush at a time, being composed of an eliminator of hair in the brush and steam to disinfect the same.

It would therefore be interesting if the art had a technical apparatus able to disinfect and sanitize more than one brush, comb and/or instruments and accessories at a time, in up to six minutes, so still preserving the handles of these brushes, combs and/or instruments and accessories.

US 2004/033182 discloses an oral hygiene device sanitizer having a flue with a heating region distal to a sterilization region, wherein an oral hygiene device chamber is located in a sterilization region. A controllable heater fixed in a flue heats up air to no more than 104 degrees Celsius which flows across an oral hygiene device (such as a toothbrush, gum stimulator or tongue scraper) for at least forty-five minutes. In another embodiment, a water chamber is positioned adjacent to a controllable heater to generate steam to be mixed with heated air and further enhance the sanitization of an oral hygiene device.

US 6 565 819 B1 discloses a housing having an upper portion, a mid portion and a lower portion, with an aperture located in the mid portion of the housing for receiving the bristle portion of a toothbrush. The upper portion of the housing has a reservoir for water, and a tube is connected for carrying selected amounts of water to a pan mounted in the lower portion of the housing. The pan is configured to contain a relatively small quantity of water delivered during a relatively short interval of time from the reservoir by means of a pump driven by an electric motor. A heater is mounted closely below the pan and arranged to heat and vaporize the small quantity of water delivered to the pan at a given moment. The motor is caused to rotate during the time of the delivery of electric current to the heater, and a timer causes the rotation of the motor to cease and the shutting off of electric current to the heater after sufficient time has elapsed for the creation of heated vapor for the sanitizing of the bristles of the toothbrush inserted through the aperture.

It is an object of the present invention to provide an apparatus for disinfecting and sanitizing brushes.

This object is achieved by the apparatus of claim 1.

In a wide way, the apparatus of the invention for disinfecting and sanitizing brushes, combs, instruments and accessories comprises one case upon a base having supports, tank for water to be vaporized, chamber for steaming, support for brushes, electric resistance heating system, protection screen, tilting lid, thermostat, electronic system of control and operation provided with electronic circuit board, transformer, fuse holder, power cable, on/off switch, blue and red LEDs and sensor.

Alternatively, the apparatus is presented in a mechanical version without using LEDs and other electronic devices.

When turning on the apparatus, the water contained in the chamber is heated, generating steam that involves the brushes. After six minutes the steam in contact with the brushes cleans thereof.

Thus, the present invention provides an apparatus for disinfecting and sanitizing brushes, the apparatus able to disinfect and sanitizes three brushes simultaneously.

The present invention also provides an apparatus for disinfecting and sanitizing brushes such that the brushes handles are not affected by the steam used for disinfecting and sanitizing.

The present invention also provides an apparatus for disinfecting and sanitizing brushes where the disinfecting and sanitizing is performed only by steam.

The present invention additionally provides an apparatus for disinfecting and sanitizing brushes, combs, instruments and accessories, the apparatus able to perform the disinfecting and sanitizing in about six minutes.

The present invention additionally provides an apparatus for disinfecting and sanitizing brushes in an electronic version.

The present invention also provides an apparatus for disinfecting and sanitizing brushes in a mechanic version.

### BRIEF DESCRIPTION OF THE FIGURES

Figure 1 illustrates an expanded right perspective view of the apparatus of the invention in the electronic version showing its component parts.

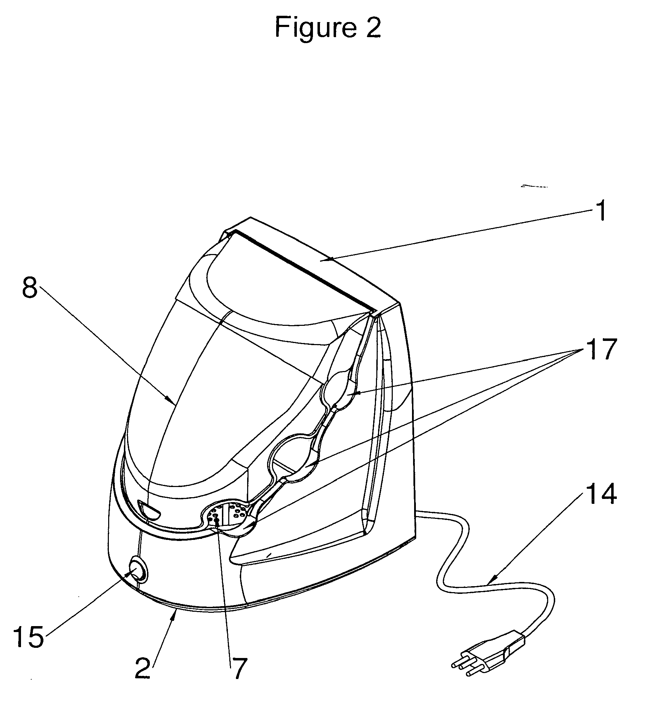

Figure 2 illustrates the apparatus of the invention in the electronic version in perspective view highlighting the right side thereof.

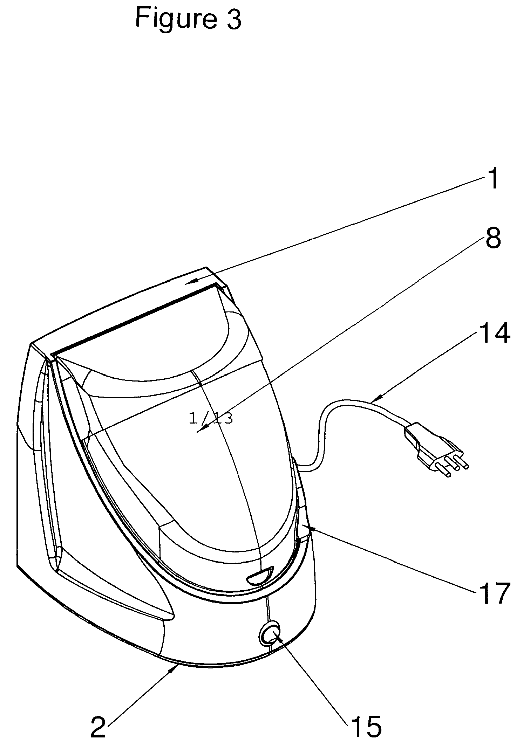

Figure 3 illustrates the apparatus of the invention in the electronic version in perspective view, highlighting the left side thereof.

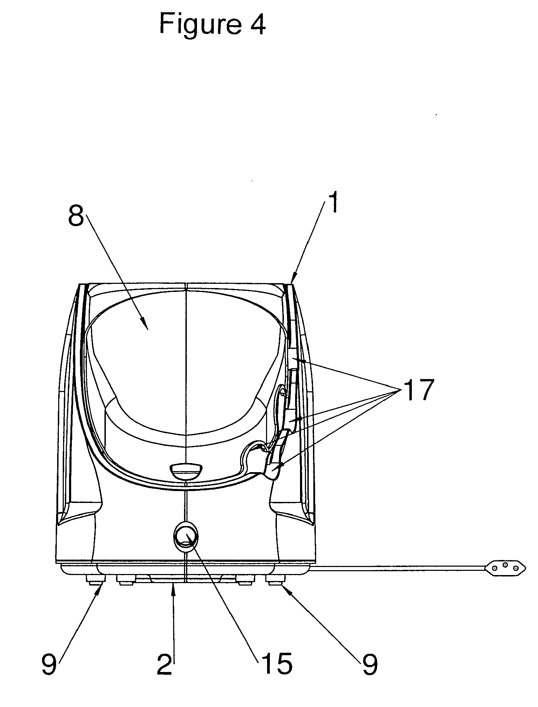

Figure 4 illustrates the apparatus of the invention in the electronic version in front view.

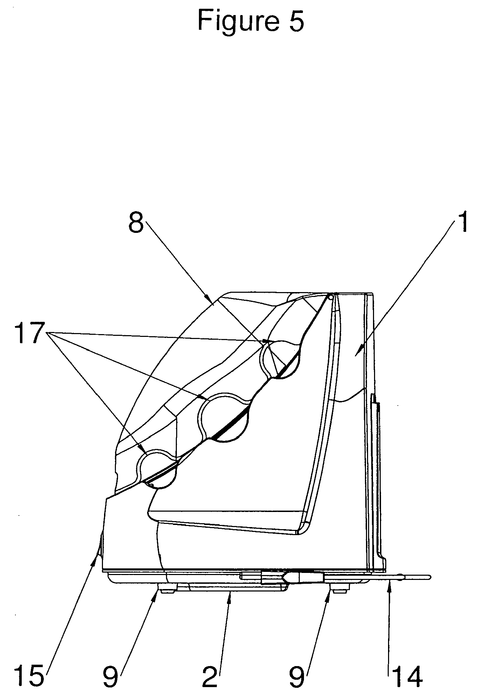

Figure 5 illustrates the apparatus of the invention in the electronic version in right side view.

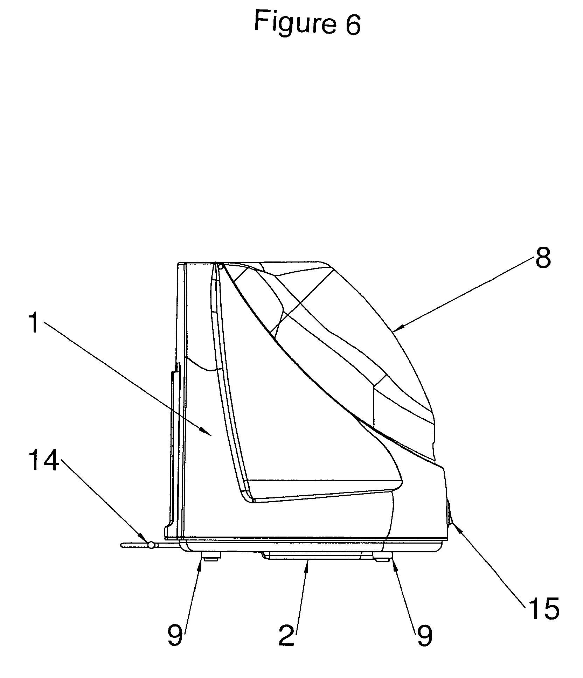

Figure 6 illustrates the apparatus of the invention in the electronic version in left side view.
Figure 7 illustrates the apparatus of the invention in the electronic version in rear view.

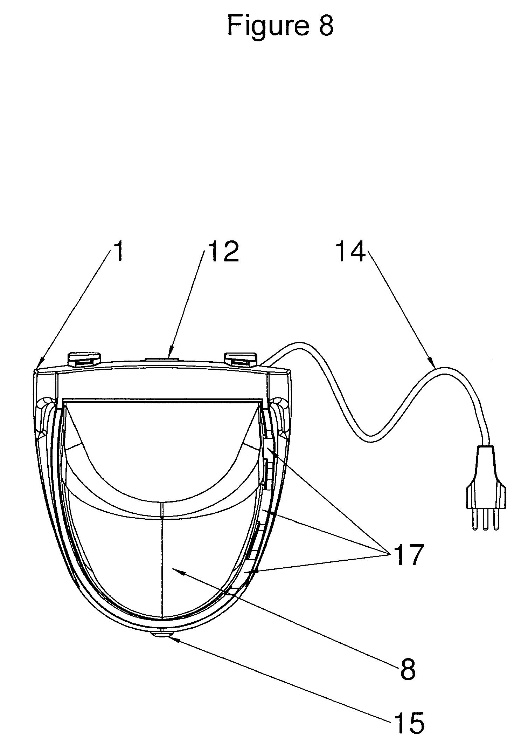

Figure 8 illustrates the apparatus of the invention in the electronic version in upper view.
Figure 9 illustrates the apparatus of the invention in the electronic version in lower view.
Figure 10 illustrates the apparatus of the invention in the electronic version in right perspective view without the lid and with the brushes properly placed in the concave fits.
Figure 11 illustrates the apparatus of the invention in the mechanic version in front view.
Figure 12 illustrates the apparatus of the invention in the mechanic version in expanded right perspective view.
Figure 13 illustrates the apparatus of the invention in the mechanic version in right perspective view.

### DETAILED DESCRIPTION OF THE PREFERRED EMBODIMENTS

According to the present invention, the term "brushes" means devices used for work in veterinary or human hair, such as brushes, combs, instruments and accessories that need to be cleaned periodically. The term thus includes any instruments such as scissors, etc., which require disinfecting and sanitizing.

The steam used for disinfecting and sanitizing is pure steam.

In order to ensure the disinfecting, products must be kept in contact with steam for four minutes.

As the apparatus takes about two minutes to reach the temperature required for steaming, the operation cycle total time is of six minutes.

Before the operation start, the operator must check if there is 100 ml of water in the tank.

Water is introduced by hand in the apparatus and refilled at each disinfecting cycle.

Brushes are inserted into the apparatus with the handles faced outside the apparatus in a horizontal position, preventing that the cables are heated and accidents occur to the user's hands.

To allow these advantages the apparatus of the invention comprises one steam chamber internally provided with a support with three concave housings, one housing for each brush or other similar instrument. The chamber, together with the case and lid, allows the said brushes of different sizes and shapes become arranged in a horizontal position in contact with the steam emanating from the chamber

The generated steam in the chamber comes into contact with the brushes to be cleaned and disinfected from bottom to top direction.

The case of the apparatus of the invention is molded in an engineering plastic, heat and impact-resistant. The case can have any color.

The case geometry of the present apparatus is not critical, but it must consider the arrangement need of the brushes and other products inside thereof such that all products receive the steam generated evenly.

An embodiment of the apparatus of the invention involves electronic controls.

Another embodiment of the apparatus involves mechanic controls.

The electronic embodiment features a control and operation electronic system and operation with electronic circuit board and sensor.

The mechanic embodiment is provided with a timer.

Both embodiments include a protection system comprising a thermostat.

The invention is described below related to the Figures attached, without, therefore, to be inferred that they are limitative of the invention. Rather, it should be clear for those skilled in the art that various modifications and variations are possible within the scope of the invention, this being enclosed only by the appended claims.

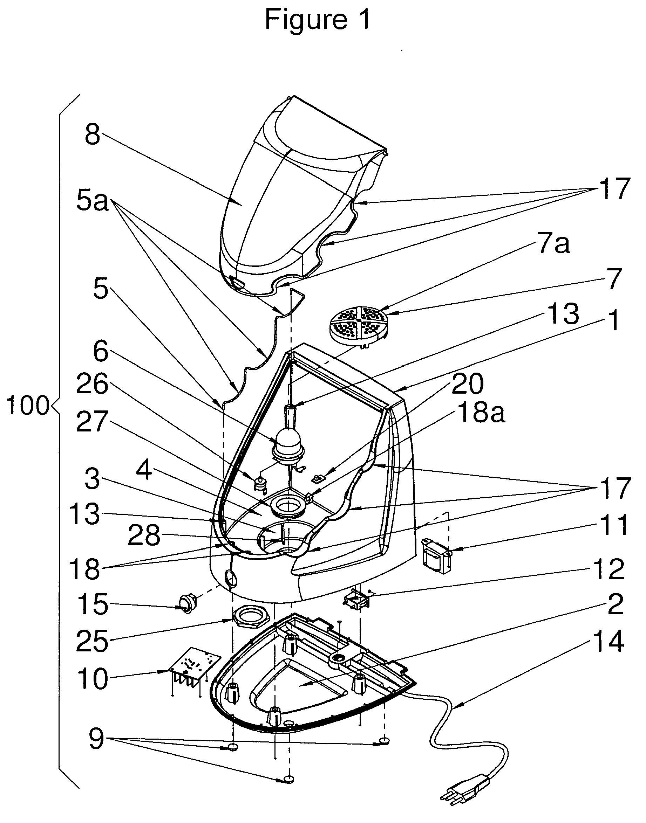

The apparatus in its electronic version, generally designed by the number (100) according to Figure 1, consists of a case (1); base (2) of the case (1) on support feet (9); water tank (3); chamber (4); support (5) for brushes (16); electric resistance heating system (6); protection screen (7); tilting lid (8); thermostat (26); control and operation electronic system provided with the electronic circuit board (10); transformer (11); fuse holder (12); power cable (14); on/off switch (15); blue LEDs (18); red LEDs (18a) and sensor (20).

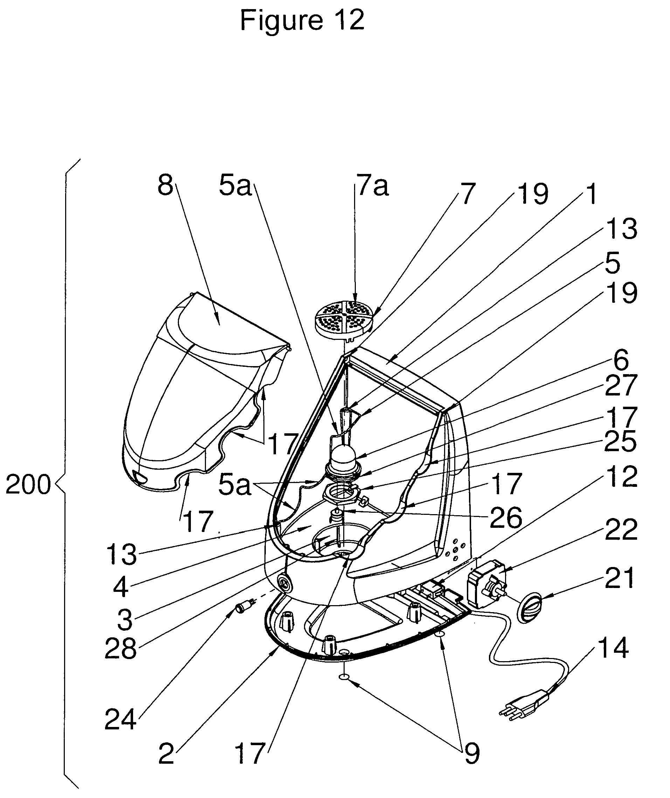

The apparatus in its mechanic version, generally designed by the number (200) (Figure 12) consists of a case (1); base (2) of the case (1); water tank (3); chamber (4); support (5) for brushes (16); electric resistance heating system (6); protection screen (7); tilting lid (8); supports (9); thermostat (26); silicon ring (27); locknut (25) of the resistance (6); timer (22) button (21); fuse holder (12); power cable (14); and, pilot lamp (24).

The apparatus, either in the electronic (100) or in the mechanic (200) version, comprises a chamber (4) with steam system. The steam is produced through the shielded resistance contact (6) with the water present in the tank (3) of the present apparatus, as can be noted in Figures 1 and 12.

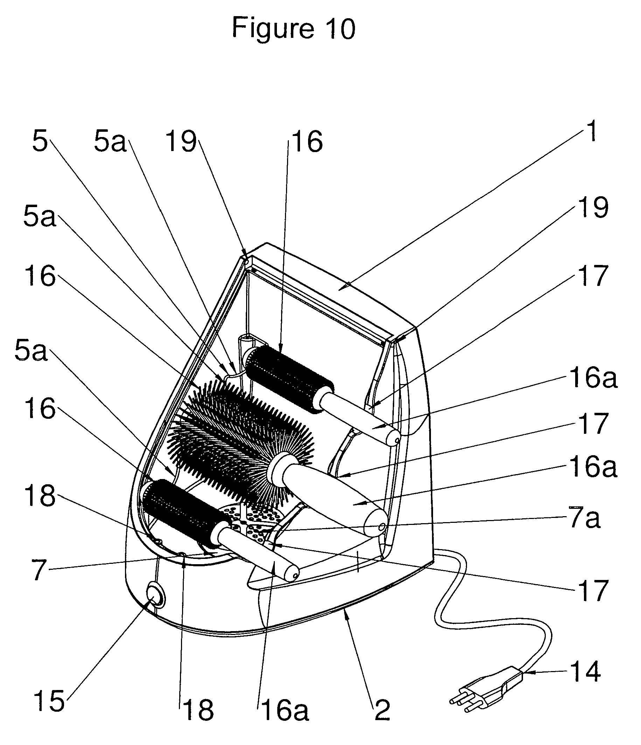

The apparatus (100, 200) allows inserting up to three brushes (16) for hair, combs, instruments and accessories, as illustrates the Figure 10. The said brushes are involved for about four minutes by the steam generated by the apparatus (100, 200) at about 75°C. This procedure eliminates the microorganisms and bacteria sensitive to the sanitizing and disinfecting process.

The support (5) for brushes body supports up to three units at the same time for each disinfecting and sanitizing cycle.

The support (5) is designed in steel or any other material that does not deteriorate with moisture, has rod-molded format with concave fits (5a) for brushes (16) handles (16a). The support (5) is fitted inside the disinfecting and sanitizing chamber (4) in housing (13).

Another support is formed with the chamber (4) itself, case (1) and tilting lid (8) of the apparatus. The support thus formed is able to sustain the handles (16a) of the products (16) object of disinfecting through semicircular or concave accommodations (17) molded in the said component parts. Thus, handles (16a) protrude outside of the apparatus (100, 200) being free of heat, thereby avoiding the possibility of accidents for the operator and maintaining handles (16a) integrity.

Brushes arrangement (16) can be noted in Figure 10.

The water tank (3) of the apparatus (100, 200) has capacity for about 100 ml of water intended to produce steam to disinfect and sanitize brushes and other products.

The tank geometry (3) is not critical, allowing circular and oval formats.

The tank base (3) is provided with fits (28) for screen insertion (7).

The screen (7) is provided with holes (7a) intended to allow a better temperature diffusion and any micro bubbles appearing on the resistance surface (6) during the tank (3) water heating.

The tilting lid (8) is transparent, in order to make visible the lighting of the LEDs (18a) intended to warn occurrence of overheating.

One way to produce steam by the apparatus (100, 200) is through an electrical resistance (6). The tank (3) has a through hole (not shown) to fit the electrical resistance (6), preferably shielded. The resistance (6) is properly stopped with a high temperature resistant silicon seal (27). The electric resistance (6) and the seal (27) are attached to each other with the aid of the nut (25).

Other stem production modes in the apparatus (100, 200) include a usual resistance or sealed (or encapsulated) resistance or by electrodes.

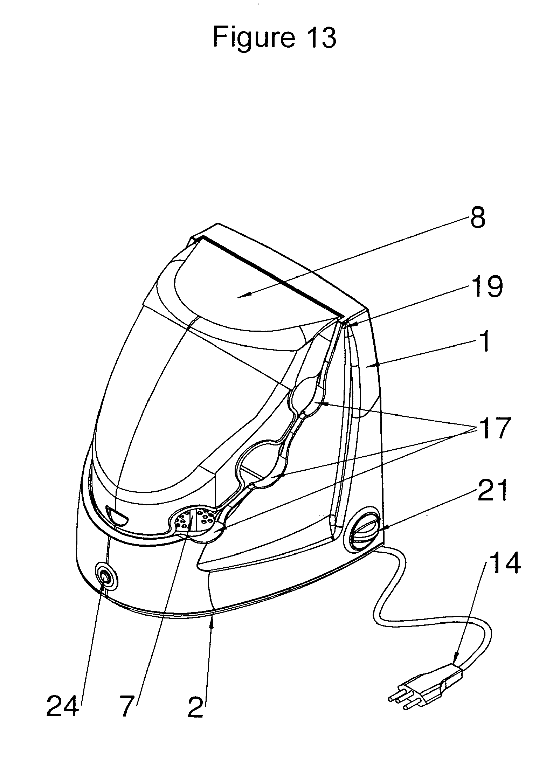

The case upper part (1) of the apparatus (100, 200) is provided with two holes (19), the said holes being responsible by the tilting lid (8) fit, as can be noted in Figures 10, 12 and 13.

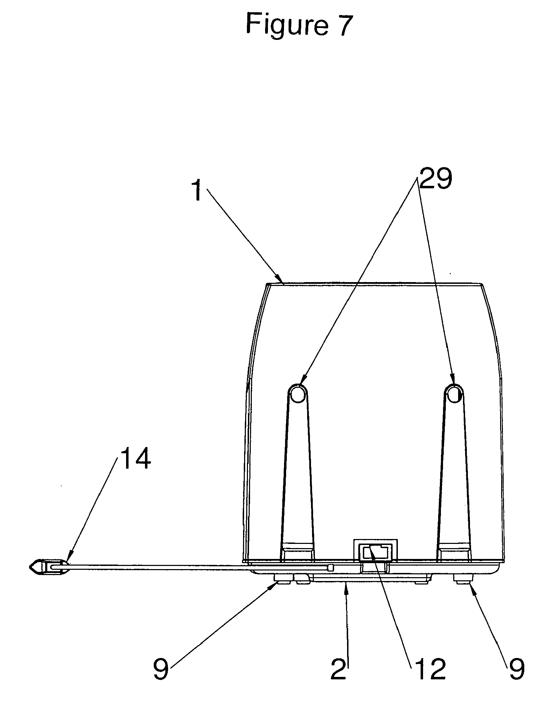

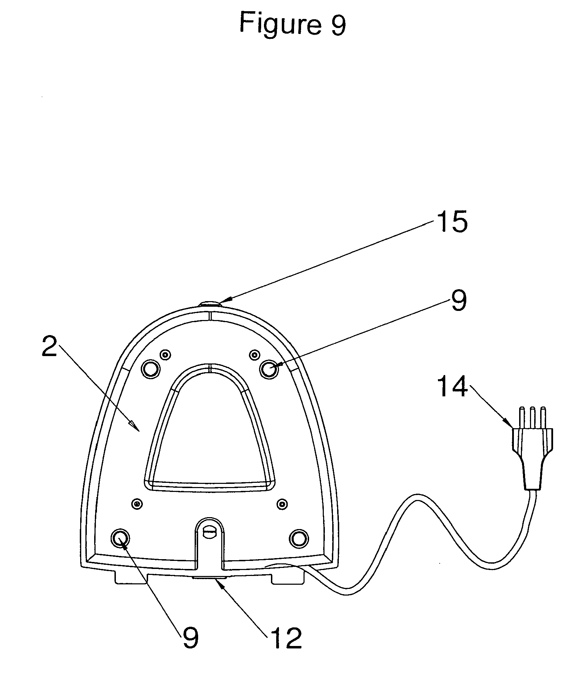

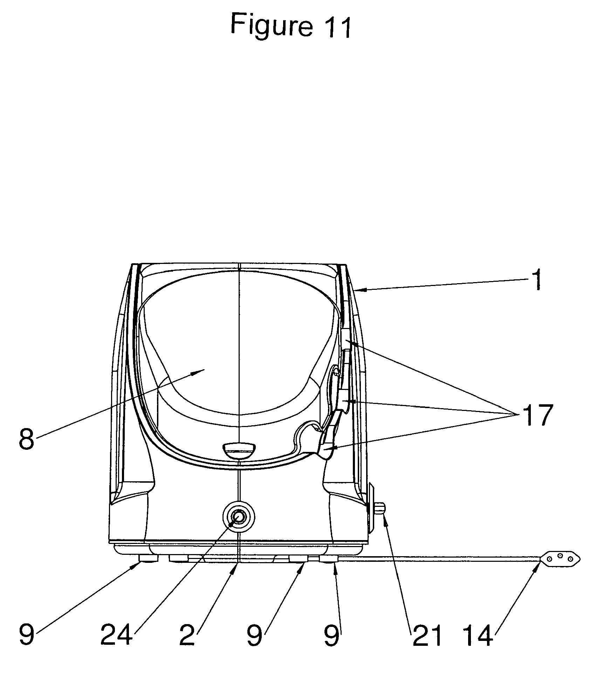

The base (2) of the apparatus (100, 200) is fitted and attached by screws in the case (1), so that the lower part of the said base is provided with rubber supports (9) without, however, being limited to this, as can be noted in Figures 7, 9 and 11.

In the electronic version (100), between the base (2) and the chamber (4) the control and operation electronic system is inserted. This system comprises electronic circuit board (10), transformer (11), fuse holder (12), power cable (14), thermostat (26), on/off switch (15), blue LEDs (18), red LEDs (18a) and sensor (20), as can be noted in Figure 1.

In the mechanic version (200), between the base (2) and the chamber (4) the control system is inserted, comprising thermostat (26), timer (22), fuse holder (12), button (21) provided with pilot lamp (24), and power cable (14), as can be noted in Figure 12.

In the rear part of the apparatus, either in the electronic (100) or in the mechanic (200) version, two holes (29) for screws fits are foreseen or hooks that provide attachment to wall, as can be noted in Figure 7.

The on/off switch (15) in the electronic version (100) is placed in the front part of the apparatus (110, 200) of the invention, so it can have a light of any color related to the on/off switch, indicating the apparatus is powered.

In the mechanic version (200) the on/off switch functionality (15) is replaced by a button (21) connected to a pilot lamp (24), as can be seen in Figure 12.

Also, in the mechanic version (200) the pilot lamp (24) replaces the LEDs (18, 18a) and the timer (22) replaces the electronic circuit board (10) of the electronic embodiment.

The pilot lamp (24) remains light up only while the timer (22) and the resistance (6) are switched on. The pilot lamp (24), the resistance (6) and the thermostat (26) are switched on in series.

Inside the chamber (4), in the electronic version (100), 2 blue LEDs (18) and 2 red LEDs (18a) are presented, as illustrates Figures 1 and 10. Through the apparatus electronic, blue LEDs (18) light up alternately indicating the disinfecting process start, and at the end of the process LEDs (18) remain lighted up.

When lacking water inside the tank (3), the apparatus, in the electronic version (100), through red LEDs (18a) alternately blinks indicating that it is necessary to add water.

Also, if the apparatus is overheated, red LEDs (18a) will light up.

Additionally in the electronic version (100) of the apparatus, in the case of: i) lack of water in the tank (3) or ii) overheating the security thermostat (26) stops the power supply to the shielded resistance (6).

The sensor (20) monitors the system temperature so the electronic circuit can manage the operation thereof. For example, if the sensor (20) notes an overheating, the electronic circuit will stop the apparatus (100) operation.

Analogous to the electronic version, also in the mechanic version (200) when missing water comes into operation the security thermostat (26), which interrupts the power supply for the shielded resistance (6)

In the case of overheating of the mechanic version (200) is equally the security thermostat (26) that stops the power supply for the shielded resistance (6).

The water supply inside the tank (3) is performed through any batcher container having 100 ml of filtered water that is discharged towards the protection screen (7). For this purpose, the lid (8) of the apparatus (100, 200), which must be empty, is opened and the water is added.

Once introduced to the apparatus (100, 200) brushes, combs or other products (16) to be disinfected and sanitized, the apparatus is switched on and the water is heated, generating steam.

The disinfecting and sanitizing of brushes, combs, instruments and accessories is started after two minutes, which is the time required for the chamber (4) of the apparatus (100, 200) reaches 75°C. After the temperature is reached, the apparatus (100, 200) takes approximately four minutes to complete the products disinfecting and sanitizing cycle.

Brushes, combs, instruments and accessories (16) are thus recovered from the apparatus (100, 200) securely for the operator and without any damage to the handles (16a) of said brushes (16) or similar products undergone to disinfecting and sanitizing.

An embodiment comprises an apparatus for disinfecting and sanitizing brushes, comprising a case )1) covered by a tilting lid (8) and supported by a base (2), and, inside the said case (1), a water tank (3), a chamber (4) with system for steaming the water contained in the tank (3), a support (5) for brushes (16), a shielded resistance heating system (6) for heating the water, a protection screen (7), a thermostat (26) and a control and operation system.

In a preferred further embodiment said system i) is inserted between the base (2) and the chamber (4) of the apparatus; and ii) comprises the electronic circuit board (10), transformer (11), fuse holder (12), power cable (14), thermostat (26), on/off switch (15), two blue LEDs (18), two red LEDs (18a) and sensor (20).In a preferred further embodiment the beginning of the disinfecting and sanitizing process is indicated by lighting up the blue LEDs (18) and the end of the process is indicated by keeping the LEDs (18) on.

In a preferred further embodiment the lack of water or overheating inside the tank (3) is indicated by alternated blinking of the red LEDs (18a).

In a preferred further embodiment in the case of i) lack of water in the tank (3) or ii) overheating the security thermostat (26) stops feeding power into the shielded resistance (6).

In a preferred further embodiment the control and operation system is electronic or mechanic.

In a preferred further embodiment, each disinfecting and sanitizing cycle of the brushes (16) uses the total amount of water contained inside the tank (3).

## Claims

1. Apparatus for disinfecting and sanitizing brushes, comprising a case (1) covered by a tilting lid (8) and supported by a base (2), and, inside the said case (1), a water tank (3), a chamber (4) with system for steaming the water contained in the tank (3), a support (5) for brushes (16), a shielded resistance heating system (6) for heating the water, a protection screen (7) fitted to a tank base, a thermostat (26) and a control and operation system,
**characterized in that** the chamber (4), together with the case (1) and the lid (8) allow the said brushes (16) to be arranged in the apparatus in a horizontal position, contacting the steam emanating from the bottom to the top of the said chamber (4), and to hold the handles (16a) of the brushes (16) through semicircular or concave accommodations (17) molded in the chamber (4), the case (1) and the lid (8) such that the handles (16a) protrude outside the apparatus and are free of heating.

2. Apparatus according to claim 1, wherein the tilting lid (8) is transparent.

3. Apparatus according to claim 1, comprising two holes (29) to fit the screws or hooks to attach in the wall.

4. Apparatus according to claim 1, wherein the steam needed to disinfect and sanitize the brushes (16) is produced through the shielded resistance contact (6) with the water in the tank (3).

5. Apparatus according to claim 1, wherein the support (5) for the brushes body (16) supports up to three units at the same time for each disinfecting and sanitizing cycle of the brushes (16).

6. Apparatus according to claim 1, wherein the support format (5) is a rod shaped with concave fits (5a) for handles (16a) of the brushes (16).

7. Apparatus according to claim 1, wherein the support (5) is fitted inside the chamber (4) in housing (13).

8. Apparatus according to claim 1, wherein the water tank base (3) is provided with fits (28) for the screen insertion (7), said screen insertion (7) being provided with holes (7a) for better temperature diffusion during the water heating of the said tank (3).

9. Apparatus according to claim 1, wherein the shielded resistance (6) is duly protected with high temperature resistant silicon seal (27).

10. Apparatus according to claim 1, wherein the control and operation system is electronic, wherein the said system i) is inserted between the base (2) and the chamber (4) of the apparatus; and ii) comprises the electronic circuit board (10), transformer (11), fuse holder (12), power cable (14), thermostat (26), on/off switch (15), two blue LEDs (18), two red LEDs (18a) and sensor (20).

11. Apparatus according to claim 1, wherein the control and operation system is mechanic, wherein the said system i) is inserted between the base (2) and the chamber (4) and ii) comprises the thermostat (26), button (21) connected to the pilot lamp (24), timer (22), fuse holder (12) and power cable (14).

12. Apparatus according to claim 1, wherein the control and operation system is mechanic, wherein the pilot lamp (24) remains on only when the timer (22) and the shielded resistance (6) are on.

13. Apparatus according to claim 1, wherein in the case of i) lack of water in the tank (3) or ii) overheating the security thermostat (26), the apparatus stops feeding power into the shielded resistance (6), wherein the pilot lamp (24), the shielded resistance (6) and the thermostat (26) are connected in series.

14. Apparatus according to claim 1, wherein the control and operation system is mechanic, wherein in the case of i) lack of water in the tank (3) or ii) overheating the security thermostat (26), the apparatus stops feeding power to the shielded resistance (6).

## Patentansprüche

1. Vorrichtung zum Desinfizieren und Sterilisieren von Bürsten, die ein Gehäuse (1), das durch einen Kippdeckel (8) abgedeckt und durch eine Basis (2) getragen wird, und im Inneren des Gehäuses (1) einen Wassertank (3), eine Kammer (4) mit einem System zum Umwandeln des in dem Tank (3) enthaltenen Wassers zu Dampf, ein Träger (5) für Bürsten (16), ein Abgeschirmter-Widerstand-Heizsystem (6) zum Erhitzen des Wassers, eine an einer Tankbasis angebrachte Schutzwand (7), ein Thermostat (26) und ein Steuer- und Betriebssystem aufweist,
**dadurch gekennzeichnet, dass** die Kammer (4) zusammen mit dem Gehäuse (1) und dem Deckel (8) ermöglicht, dass die Bürsten (16) in einer horizontalen Position in der Vorrichtung angeordnet werden, wobei sie mit dem Dampf in Kontakt kommen, der von dem unteren bis zu dem oberen Ende der Kammer (4) austritt, und dass die Griffe (16a) der Bürsten (16) durch halbkreisförmige oder konkave Aufnahmen (17), die in der Kammer (4), in dem Gehäuse (1) und in dem Deckel (8) gebildet sind, hindurch derart zu halten, dass die Griffe (16a) außerhalb der Vorrichtung vorstehen und nicht erhitzt werden.

2. Vorrichtung gemäß Anspruch 1, bei der der Kippdeckel (8) durchsichtig ist.

3. Vorrichtung gemäß Anspruch 1, die zwei Löcher (29) aufweist, um die Schrauben oder Haken, die in der Wand zu befestigen sind, anzubringen.

4. Vorrichtung gemäß Anspruch 1, bei der der zum Desinfizieren und Sterilisieren der Bürsten (16) benötigte Dampf durch den Abgeschirmter-Widerstand-Kontakt (6) mit dem Wasser in dem Tank (3) erzeugt wird.

5. Vorrichtung gemäß Anspruch 1, bei der der Träger (5) für den Bürstenkörper (16) gleichzeitig bis zu drei Einheiten für jeden Desinfektions- und Sterilisationszyklus der Bürsten (16) trägt.

6. Vorrichtung gemäß Anspruch 1, bei der das Trägerformat (5) eine Stange ist, die mit konkaven Passungen (5a) für Griffe (16a) der Bürsten (16) geformt ist.

7. Vorrichtung gemäß Anspruch 1, bei der der Träger (5) im Inneren der Kammer (4) in dem Gehäuse (13) angebracht ist.

8. Vorrichtung gemäß Anspruch 1, bei der die Wassertankbasis (3) mit Passungen (28) für die Wandeinfügung (7) versehen ist, wobei die Wandeinfügung (7) mit Löchern (7a) zum Zweck einer besseren Temperaturverteilung während des Erhitzens des Wassers des Tanks (3) versehen ist.

9. Vorrichtung gemäß Anspruch 1, bei der der abgeschirmte Widerstand (6) mit einer hochtemperaturbeständigen Silikonabdichtung (27) gebührend geschützt ist.

10. Vorrichtung gemäß Anspruch 1, bei der das Steuer- und Betriebssystem elektronisch ist, wobei das System i) zwischen die Basis (2) und die Kammer (4) der Vorrichtung eingefügt ist; und ii) die elektronische Schaltungsplatine (10), den Transformator (11), den Sicherungshalter (12), das Netzkabel (14), den Thermostat (26), den Ein-/Aus-Schalter (15), zwei blaue LEDs (18), zwei rote LEDs (18a) und den Sensor (20) aufweist.

11. Vorrichtung gemäß Anspruch 1, bei der das Steuer- und Betriebssystem mechanisch ist, wobei das System i) zwischen die Basis (2) und die Kammer (4) eingefügt ist und ii) den Thermostat (26), den mit der Kontrolllampe (24) verbundenen Knopf (21), den Zeitgeber (22), den Sicherungshalter (12) und das Netzkabel (14) aufweist.

12. Vorrichtung gemäß Anspruch 1, bei der das Steuer- und Betriebssystem mechanisch ist, wobei die Kontrolllampe (24) nur dann eingeschaltet bleibt, wenn der Zeitgeber (22) und der abgeschirmte Widerstand (6) eingeschaltet sind.

13. Vorrichtung gemäß Anspruch 1, bei der in dem Fall i) von fehlendem Wasser in dem Tank (3) oder ii) einer Überhitzung des Sicherheitsthermostats (26) die Vorrichtung ein Zuführen von Leistung in den abgeschirmten Widerstand (6) anhält, wobei die Kontrolllampe (24), der abgeschirmte Widerstand (6) und das Thermostat (26) in Reihe geschaltet sind.

14. Vorrichtung gemäß Anspruch 1, bei der das Steuer- und Betriebssystem mechanisch ist, wobei in dem Fall i) von fehlendem Wasser in dem Tank (3) oder ii) einer Überhitzung des Sicherheitsthermostats (26) die Vorrichtung ein Zuführen von Leistung zu dem abgeschirmten Widerstand (6) anhält

## Revendications

1. Appareil pour la désinfection et l'hygiénisation de brosses, comprenant un boîtier (1) couvert par un couvercle basculant (8) et supporté par une base (2) et, à l'intérieur dudit boîtier (1), un réservoir d'eau (3), une chambre (4) avec un système pour la vaporisation de l'eau contenue dans le réservoir (3), un support (5) pour brosses (16), un système de chauffage par résistance blindée (6) pour chauffer l'eau, un écran de protection (7) monté sur une base de réservoir, un thermostat (26) et un système de commande et d'actionnement,
**caractérisé par le fait que** la chambre (4) ensemble avec le boîtier (1) et le couvercle (8) permettent que lesdites brosses (16) soient disposées dans l'appareil en position horizontale de manière à entrer en contact avec la vapeur émanant du bas vers le haut de ladite chambre (4) et de maintenir les poignées (16a) des brosses (16) à travers des logements semi-circulaires ou concaves (17) moulés dans la chambre (4), le boîtier (1) et le couvercle (8) de sorte que les poignées (16a) ressortent à l'extérieur de l'appareil et ne soient pas chauffées.

2. Appareil selon la revendication 1, dans lequel le couvercle basculant (8) est transparent.

3. Appareil selon la revendication 1, comprenant deux trous (29) pour monter les vis ou crochets à fixer dans le mur.

4. Appareil selon la revendication 1, dans lequel la vapeur nécessaire pour la désinfection et l'hygiénisation des brosses (16) est produite par le contact de la résistance blindée (6) avec l'eau dans le réservoir (3).

5. Appareil selon la revendication 1, dans lequel le support (5) pour le corps de brosse (16) supporte jusqu'à trois unités en même temps pour chaque cycle de désinfection et d'hygiénisation des brosses (16).

6. Appareil selon la revendication 1, dans lequel le format de support (5) est une tige formée avec des ajustements concaves (5a) pour les poignées (16a) des brosses (16).

7. Appareil selon la revendication 1, dans lequel le support (5) est monté à l'intérieur de la chambre (4) dans le boîtier (13).

8. Appareil selon la revendication 1, dans lequel la base du réservoir d'eau (3) est pourvue d'ajustements (28) pour l'insertion de l'écran (7), ladite insertion d'écran (7) étant pourvue de trous (7a) pour une meilleure diffusion de la température pendant le chauffage de l'eau dudit réservoir (3).

9. Appareil selon la revendication 1, dans lequel la résistance blindée (6) est dûment protégée par un joint de silicone résistant à haute température (27).

10. Appareil selon la revendication 1, dans lequel le système de commande et d'actionnement est électronique, dans lequel ledit système i) est inséré entre la base (2) et la chambre (4) de l'appareil; et ii) comprend la carte de circuit électronique (10), le transformateur (11), le porte-fusible (12), le câble d'alimentation (14), le thermostat (26), l'interrupteur marche/arrêt (15), deux LED bleues (18), deux LED rouges (18a) et le capteur (20).

11. Appareil selon la revendication 1, dans lequel le système de commande et d'actionnement est mécanique, dans lequel ledit système i) est inséré entre la base (2) et la chambre (4) et ii) comprend le thermostat (26), le bouton (21) connecté à la lampe témoin (24), la minuterie (22), le porte-fusible (12) et le câble d'alimentation (14).

12. Appareil selon la revendication 1, dans lequel le système de commande et d'actionnement est mécanique, dans lequel la lampe témoin (24) ne reste allumée que lorsque la minuterie (22) et la résistance blindée (6) sont activées.

13. Appareil selon la revendication 1, dans lequel, en cas de i) manque d'eau dans le réservoir (3) ou ii) une surchauffe du thermostat de sécurité (26), l'appareil arrête l'alimentation de courant vers la résistance blindée (6), dans lequel le lampe témoin (24), la résistance blindée (6) et le thermostat (26) sont connectés en série.

14. Appareil selon la revendication 1, dans lequel le système de commande et d'actionnement est mécanique, dans lequel, en cas de i) manque d'eau dans le réservoir (3) ou ii) une surchauffe du thermostat de sécurité (26), l'appareil arrête l'alimentation de courant vers la résistance blindée (6).
